(19) Europäisches Patentamt
European Patent Office
Office européen des brevets

(11) EP 2 196 188 A2

(12) **DEMANDE DE BREVET EUROPEEN**

(43) Date de publication:
**16.06.2010 Bulletin 2010/24**

(21) Numéro de dépôt: **09178924.8**

(22) Date de dépôt: **11.12.2009**

(51) Int Cl.:
***A61K 8/49*** *(2006.01)* ***A61Q 5/10*** *(2006.01)*
***A61K 8/19*** *(2006.01)* ***A61K 8/22*** *(2006.01)*
***A61K 8/27*** *(2006.01)*

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **12.12.2008 FR 0858556**

(71) Demandeur: **L'Oréal**
**75008 Paris (FR)**

(72) Inventeurs:
- **Guerin, Frédéric**
  **75010, Paris (FR)**
- **Gourlaouen, Luc**
  **92600, Asnieres (FR)**

(74) Mandataire: **Fevrier, Murielle Françoise E.**
**L'Oréal**
**River Plaza - DIPI**
**25-29 Quai Aulagnier**
**92665 Asnières-sur-Seine (FR)**

(54) **Procédé de coloration capillaire mettant en oeuvre un dérivé de l'hématoxyline, de l'hématéine, de la braziline ou de la braziléine, un sel métallique, du péroxyde d'hydrogène et du (bi)carbonate**

(57) L'invention a pour objet un procédé de coloration de fibres kératiniques par traitement desdites fibres avec i) au moins de l'hématoxyline et/ou de l'hématéine et/ou de la braziline et/ou de la braziléine, ii) au moins un sel métallique, iii) au moins du peroxyde d'hydrogène ou au moins un système générateur de peroxyde d'hydrogène et iv) au moins du (bi)carbonate.

Un autre objet de l'invention est un dispositif à plusieurs compartiments comprenant les ingrédients i), ii) et iii) et iv).

Ce procédé de coloration capillaire permet d'obtenir de meilleures colorations plus homogènes, chromatiques, tenaces et qui n'altèrent pas les propriétés cosmétiques des fibres kératiniques à partir d'extrait l'hématoxyline et/ou de l'hématéine et/ou de la braziline et/ou de la braziléine notamment naturels.

EP 2 196 188 A2

## Description

L'invention a pour objet un procédé de coloration de fibres kératiniques par traitement desdites fibres avec i) au moins de l'hématoxyline et/ou de l'hématéine et/ou de la braziline et/ou de la braziléine, ii) au moins un sel métallique, iii) au moins du peroxyde d'hydrogène ou au moins un système générateur de peroxyde d'hydrogène et iv) au moins du (bi)carbonate.

Il est connu d'obtenir des colorations dites « permanentes » avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. On sait également que l'on peut faire varier les nuances obtenues en associant ces bases d'oxydation à des coupleurs ou modificateurs de coloration, ces derniers étant choisis notamment parmi les méta-diamines aromatiques, les méta-aminophénols, les méta-diphénols et certains composés hétérocycliques tels que des composés indoliques. Ce procédé de coloration d'oxydation consiste à appliquer sur les fibres kératiniques, des bases ou un mélange de bases et de coupleurs avec du peroxyde d'hydrogène ($H_2O_2$ ou eau oxygénée) à titre d'agent oxydant, à laisser diffuser, puis à rincer les fibres. Les colorations qui en résultent sont permanentes, puissantes, et résistantes aux agents extérieurs, notamment à la lumière, aux intempéries, aux lavages, à la transpiration et aux frottements.

Cependant les colorations capillaires commerciales qui les contiennent peuvent présenter des inconvénients comme le tachage, les problèmes d'odeur, de confort et de dégradation des fibres kératiniques. C'est particulièrement le cas avec les colorations d'oxydation.

Dans le domaine de la coloration, il est également connu de colorer des matières kératiniques telles que les cheveux ou la peau à partir de composés orthodiphénols en présence d'un sel métallique notamment de Mn et/ou de Zn. En particulier les demandes de brevets FR 2 814 943, FR 2 814 945, FR 2 814 946, FR 2 814 947, proposent des compositions pour la coloration de la peau ou des fibres kératiniques, comprenant un précurseur de colorant qui contient au moins un orthodiphénol, des sels et oxydes de Mn et/ou Zn, des alcalins de type hydrogénocarbonate dans un ratio particulier Mn, Zn / hydrogénocarbonate et éventuellement une enzyme. Selon ces documents il est possible d'obtenir des colorations intenses en s'affranchissant du peroxyde d'hydrogène. Cependant les colorations obtenues sont insuffisamment intenses notamment dans le cas des fibres capillaires.

Il existe un besoin de développer des procédés de colorations qui permettent d'obtenir des colorations puissantes à partir de colorants ou de précurseurs de colorants particuliers, tout en limitant la décoloration des fibres kératiniques. En particulier, il existe un besoin d'obtenir des colorations moins agressives pour les cheveux et dans un même temps qui résistent aux agents extérieurs (lumière, intempéries, shampooings), qui soit tenace et homogène tout en restant puissante et chromatique.

Ce but est atteint par la présente invention qui a pour objet un procédé de coloration des fibres kératiniques, dans lequel lesdites fibres sont traitées par :

i) un ou plusieurs composé(s), synthétique(s) ou naturel(s), seuls ou en mélanges, choisis parmi les composés de formule (I) et (II) et leurs formes mésomères, leurs stéréoisomères, leurs sels d'addition à un acide ou de base cosmétiquement acceptable, ainsi que les hydrates ;

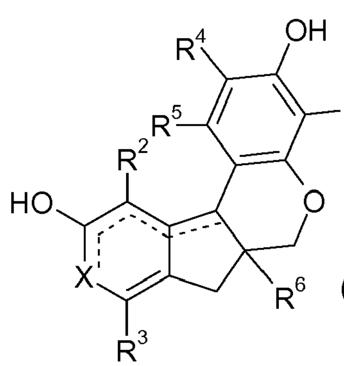 **(I)** 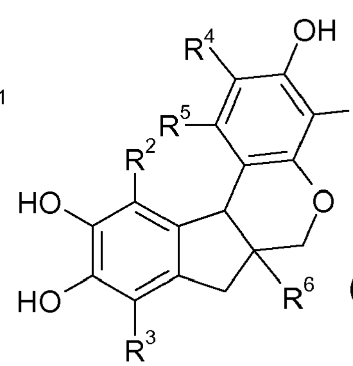 **(II)**

formules **(I)** et **(II)** dans lesquelles :

- ➢ ---- représente une liaison carbone - carbone simple ou double conjuguée,
- ➢ X représente un groupement :

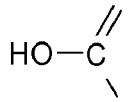 ou 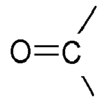

> ➢ $R^1$, $R^2$, $R^3$, $R^4$, $R^5$ et $R^6$, identiques ou différents, représentent un atome d'hydrogène, un groupe hydroxy, un groupe alkyle éventuellement substitué, alcoxy éventuellement substitué, ou un groupe acyloxy éventuellement substitué

ii) un ou plusieurs sel(s) métallique(s) ;
iii) du peroxyde d'hydrogène ou un ou plusieurs système(s) générateur(s) de peroxyde d'hydrogène ; et
iv) un ou plusieurs (bi)carbonate(s).

L'invention a également pour objet une composition cosmétique pour la teinture de fibres kératiniques comprenant les ingrédients i) à iv) tels que définis précédemment.
Un autre objet de l'invention concerne un dispositif à plusieurs compartiments comprenant les ingrédients i) à iv) tels que définis précédemment.
Le procédé selon l'invention présente l'avantage de colorer les fibres kératiniques notamment humaines, avec des résultats de colorations puissantes, chromatiques, résistantes aux lavages, la transpiration, le sébum et à la lumière et de plus durables sans altération desdites fibres. De plus les colorations obtenues à partir du procédé donnent des couleurs homogènes de la racine à la pointe d'une fibre (faible sélectivité de coloration).

*i) les composés de formules **(I)** ou **(II)** ;*

Les composés de formule (I) tels que définis précédemment peuvent se trouver sous deux formes mésomères notées (Ia) et (Ib) :

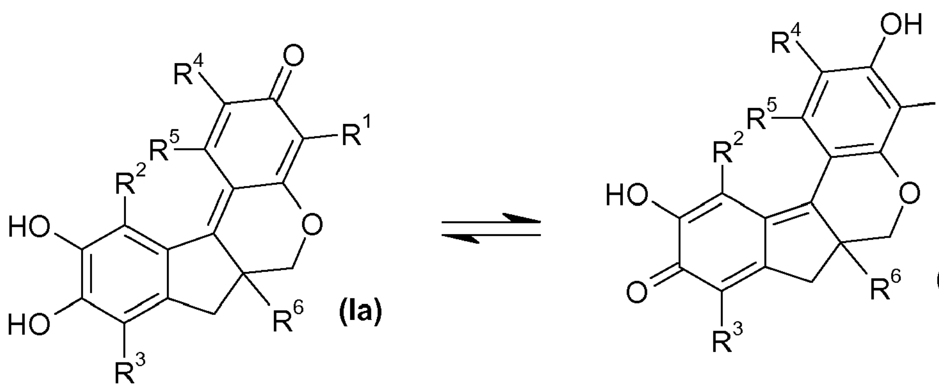

**(Ia)** **(Ib)**

Les radicaux alkyles sont des radicaux hydrocarbonés, saturés, linéaires ou ramifiés, généralement en $C_1$-$C_{20}$, particulièrement en $C_1$-$C_{10}$, de préférence les radicaux alkyles en $C_1$-$C_6$, tels que méthyle, éthyle, propyle, butyle, pentyle et hexyle.
Les radicaux alcoxy sont des radicaux alkyle-oxy avec alkyle tel que défini précédemment, en général les radicaux alcoxy en $C_1$-$C_{10}$, tels que méthoxy, éthoxy, propoxy et butoxy.
Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.
Les radicaux alkyles ou alkoxy lorsqu'ils sont éventuellement substitués, peuvent être substitués par au moins un substituant porté par au moins un atome de carbone, choisi parmi :

- un atome d'halogène;
- un groupement hydroxy ;
- un radical alkoxy en $C_1$-$C_2$ ;
- un radical alcoxycarbonyle en $C_1$-$C_{10}$ ;
- un radical (poly)-hydroxyalkoxy en $C_2$-$C_4$ ;
- un radical amino ;
- un radical héterocycloalkyle à 5 ou 6 chaînons ;

- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  * un groupement hydroxy,
  * un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
  * un groupement ammonium quaternaire $-N^+R'R''R'''$, $M^-$ pour lequel R', R", R"', identiques ou différents représentent un atome d'hydrogène, ou un groupement alkyle en $C_1$-$C_4$; et $M^-$ représente le contre-ion de l'acide organique, minéral ou de l'halogénure correspondant,
  * ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N-SO_2$-) dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- un groupement cyano ;
- un groupement nitro ;
- un groupement carboxy ou glycosylcarbonyle ;
- un groupement phénylcarbonyloxy éventuellement substitué par un ou plusieurs groupements hydroxy ;
- un groupement glycosyloxy ; et
- un groupement phényle éventuellement substitué par un ou plusieurs groupements hydroxy.

Par radical glycosyle on entend un radical issu d'un mono ou polysacharride.

Les composés de formule (I) utiles dans le procédé de l'invention peuvent être naturels ou synthétiques. Parmi les composés naturels on inclut les composés qui sont présents dans la nature et qui peuvent être reproduits par synthèse chimique.

Les sels des composés de formule (I) de l'invention peuvent être des sels d'acides ou de bases cosmétiquement acceptables. Les acides peuvent être minéraux ou organiques. De préférence l'acide est l'acide chlorhydrique qui conduit aux chlorures.

Les bases peuvent être minérales ou organiques. Particulièrement les bases sont des hydroxydes alcalins tels que la soude qui conduit à des sels de sodium.

Selon un mode particulier de l'invention les composés de formule (I) ou (II) comprennent un radical $R^6$ qui représente un groupe hydroxy.

Un autre mode particulier de l'invention concerne les composés de formule (I) ou (II) pour lesquels $R^1$ représente un atome d'hydrogène ou un groupe hydroxy.

Un mode particulier de l'invention concerne les composés préférentiellement les composés de formule (I).

Plus particulièrement le procédé de coloration des fibres kératiniques met en oeuvre comme ingrédient i) un ou plusieurs composés choisis parmi les composés naturels d'hématoxyline, d'hématéine, de braziline et de braziléine.

Parmi les composés d'hématoxyline/hématéine et de braziline/braziléine on peut citer à titre d'exemple l'hématoxyline (Natural Black 1) et la braziline (Natural Red 24), composés de la famille des indochromanes, qui sont accessibles dans le commerce. Ces derniers peuvent exister sous une forme oxydée et être obtenus par voies de synthèse ou voies d'extraction de plantes ou végétaux connus pour être riches en ces composés.

| | |
|---|---|
| 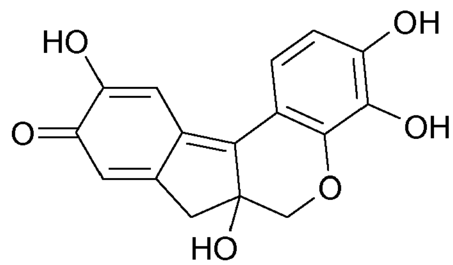<br>**Hématéine (forme oxydée) appartenant à la formule (I)** | 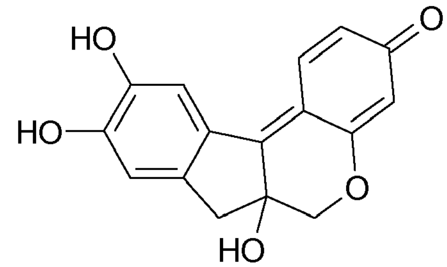<br>**Braziléine (forme oxydée) appartenant à la formule (I)** |
| 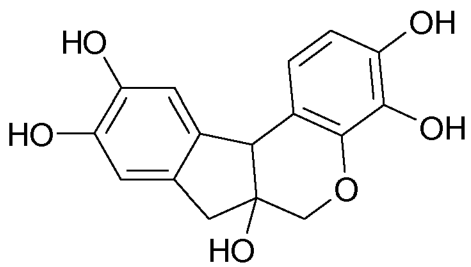<br>**Hématoxyline (Natural Black 1 - CAS 517-28-2) appartenant à la formule (II)** | 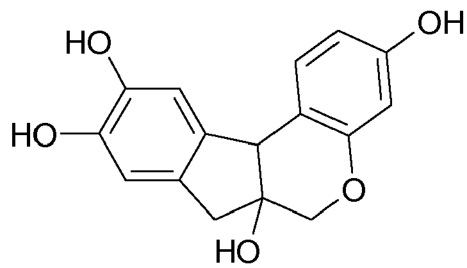<br>**Braziline (Natural Red 24 - CAS 474-07-7) appartenant à la formule (II)** |

Les composés de formule (I) et (II) peuvent être utilisés sous forme d'extraits. On peut utiliser les extraits végétaux suivants (genre et espèces) : *Haematoxylon campechianum, Haematoxylon brasiletto, Caesalpinia echinata, Caesalpinia sappan, Caesalpinia spinosa, et Caesalpina Brasiliensis*

Les extraits sont obtenus par extraction de diverses parties de plantes telles que par exemple la racine, le bois, l'écorce, la feuille,

Selon un mode de réalisation les composés d'hématoxyline/hématéine et de braziline/braziléine naturels sont issus des bois de campêches ou du brésil

De préférence le ou les composés de formule (I) ou (II) naturels de l'invention sont issus d'extraits de plantes.

Particulièrement l'ingrédient i) représente au moins 80 % en poids par rapport au poids total des ingrédients i), ii), iii) et iv) ou i), ii) et iii) tels que définis précédemment.

On peut également utiliser des mélanges d'extraits végétaux.

Les extraits naturels selon l'invention peuvent se présenter sous forme de poudres ou de liquides. De préférence les extraits de l'invention se présentent sous forme des poudres.

Selon l'invention, le ou les composé(s) de formule (I) et (II), synthétique(s), naturel(s), tels que les composés d'hématoxyline/hématéine et de braziline/braziléine et/ou le ou les extrait(s) naturel(s) utilisé(s) comme ingrédient i) dans une ou plusieurs composition(s) utile(s) dans le procédé selon l'invention représente(nt) de préférence, de 0.001% à 20% en poids du poids total de la ou des compositions contenant le ou les composés de formule (I) et (II) tels que les composés d'hématoxyline/hématéine et de braziline/braziléine ou le ou les extraits.

En ce qui concerne les composés de formule (I) et (II) purs tels que les composés d'hématoxyline/hématéine et de braziline/braziléine,purs la teneur dans la ou les compositions les contenant est comprise de préférence entre 0,001 et 5 % en poids de chacune de ces compositions.

En ce qui concerne les extraits, la teneur dans la ou les compositions contenant les extraits tels quels est comprise de préférence entre 0,5 et 20 % en poids de chacune de ces compositions.

*ii) sel métallique.*

Le procédé de l'invention utilise un ou plusieurs ingrédient(s) ii) qui sont des sel(s) métallique(s).

Particulièrement les sels métalliques sont choisis parmi les sels de magnésium (Mn) et de zinc (Zn).

Au sens de la présente invention on entend par sels, les oxydes de ces métaux et les sels proprement dits issus de l'action d'un acide sur un métal. De préférence les sels ne sont pas des oxydes. Parmi les sels on peut citer les halogénures tels que les chlorures, fluorures et iodures ; les sulfates, les phosphates ; les nitrates ; les perchlorates et les sels d'acides carboxyliques et les sels polymériques ainsi que leurs mélanges.

Plus particulièrement le sel de manganèse est différent du carbonate de manganèse, de l'hydrogénocarbonate de manganèse ou du dihydrogénocarbonate de manganèse.

Les sels d'acides carboxyliques utilisables dans l'invention incluent également des sels d'acides carboxyliques hydroxylés tels que le gluconate.

A titre d'exemple de sels polymériques on peut citer la pyrrolidone carboxylate de manganèse.

A titre d'exemple, on peut citer le chlorure de manganèse, le fluorure de Mn, l'acétate de Mn tétrahydraté, le lactate de Mn trihydraté, le phosphate de Mn, l'iodure de Mn, le nitrate de Mn trihydraté, le bromure de Mn, le perchlorate de Mn tétrahydraté, le sulfate de Mn monohydraté et le gluconate de manganèse. Les sels utilisés avantageusement sont le gluconate de manganèse et le chlorure de manganèse.

Parmi les sels de zinc on peut citer le sulfate de zinc, le gluconate de zinc, le chlorure de zinc, le lactate de zinc, l'acétate de zinc, le glycinate de zinc et l'aspartate de zinc.

Les sels de Mn et Zn peuvent être introduits sous forme solide dans les compositions ou bien provenir d'une eau naturelle, minérale ou thermale, riche en ces ions ou encore d'eau de mer (mer morte notamment). Ils peuvent aussi provenir de composés minéraux comme terres, les ocres comme les argiles (argile verte par exemple) ou même d'extrait végétal les contenant (cf. par exemple brevet le document FR 2 814 943).

Particulièrement les sels métalliques de l'invention sont de degrés d'oxydation 2 tels que le Mn(II) et le Zn(II).

Selon un mode de réalisation préféré de l'invention, le ou les sels métalliques utilisés représentent de 0.001% à 0,1% en poids environ du poids total de la ou des composition(s) contenant ce ou ces sels métalliques et encore plus préférentiellement de 0,05% à 10% en poids environ.

*iii) peroxyde d'hydrogène ou système générateur de peroxyde d'hydrogène*

Dans le cadre de la présente invention, le troisième constituant est iii) du peroxyde d'hydrogène ou un système générateur de peroxyde d'hydrogène tel que :

a) le peroxyde d'urée ;
b) les complexes polymériques pouvant libérer du peroxyde d'hydrogène tels que le polyvinylpyrrolidone/$H_2O_2$ en particulier se présentant sous forme de poudres et les autres complexes polymériques décrits dans US 5,008,093 ; US 3,376,110 ; US 5,183,901 ;
c) les oxydases produisant du peroxyde d'hydrogène en présence d'un substrat adéquat (par exemple le glucose dans le cas de glucose oxydase ou l'acide urique avec l'uricase) ;
d) les peroxydes de métaux générant dans l'eau du peroxyde d'hydrogène comme le peroxyde de calcium, peroxyde de magnésium;
e) les perborates ; ou
f) les percarborates.

Selon un mode de réalisation préféré de l'invention la composition contient un ou plusieurs système(s) générateur(s) de peroxyde d'hydrogène choisi(s) parmi a) le peroxyde d'urée, b) les complexes polymériques pouvant libérer du peroxyde d'hydrogène choisis parmi le polyvinylpyrrolidone/$H_2O_2$; c) les oxydases ; e) les pérborates et f) le percarborates.

Particulièrement le troisième constituant est du peroxyde d'hydrogène ou du peroxyde d'urée. Préférentiellement du peroxyde d'hydrogène.

Par ailleurs la ou les compositions comprenant le peroxyde d'hydrogène ou le générateur de peroxyde d'hydrogène peut également renfermer divers adjuvants utilisés classiquement dans les compositions pour la teinture des cheveux et tels que définis ci-après au point vi).

Selon un mode particulier de l'invention, le peroxyde d'hydrogène ou le ou les système(s) générateur(s) de peroxyde d'hydrogène utilisé représente de préférence, de 0.001% à 12% en poids de peroxyde d'hydrogène par rapport au poids total de la composition ou des compositions le ou les contenant et encore plus préférentiellement de 0,2% à 2,7% en poids.

*iv) (bi)carbonate ou système générateur de (bi)carbonate(s)*

Dans le cadre de la présente invention, le quatrième ingrédient choisi parmi les (bi)carbonates.

Par (bi)carbonates est sous entendu :

a) les carbonates de métal alcalins ($Mét_2^+$, $CO_3^{2-}$), de métal alcalino-terreux ($Mét'^{2+}$, $CO_3^{2-}$) d'ammonium ($(R''_4N^+)_2$, $CO_3^{2-}$) ou de phosphonium ($(R''_4P^+)_2$,$CO_3^{2-}$ avec Mét' représentant un métal alcalino-terreux et Mét représentant un métal alcalin, et R'', identiques ou différents, représentent un atome d'hydrogène, un groupement ($C_1$-$C_6$)alkyle

éventuellement substitué tel que hydroxyéthyle),
et
b) les bicarbonates, également appelés hydrogénocarbonates, de formules suivantes :

> ➢ $R'^{+}$, $HCO_3^-$ avec R' représentant un atome d'hydrogène, un métal alcalin, un groupement ammonium $R''_4N^+$- ou phosphonium $R''_4P^+$- où R", identiques ou différents, représentent un atome d'hydrogène, un groupement ($C_1$-$C_6$)alkyle éventuellement substitué tel qu'hydroxyéthyle et lorsque R' représente un atome d'hydrogène l'hydrogénocarbonate est alors appelé dihydrogénocarbonate ($CO_2$, $H_2O$) ; et
> ➢ $Mét'^{2+}$ $(HCO_3^-)_2$ avec Mét' représentant un métal alcalino-terreux.

Plus particulièrement, le quatrième ingrédient est choisi parmi les (bi)carbonates de métal alcalin ou alcalino-terreux ; préférentiellement les (bi)carbonates de métal alcalin.
On peut citer les carbonates ou hydrogénocarbonates de Na, K, Mg, Ca et leurs mélanges, et en particulier l'hydrogénocarbonate de Na. Ces hydrogénocarbonates peuvent provenir d'une eau naturelle, par exemple eau de source du bassin de Vichy, de La Roche Posay, eau de Badoit (cf. brevet par exemple le document FR 2 814 943). Particulièrement on peut citer le carbonate de sodium [497-19-8] = $Na_2CO_3$, l'hydrogénocarbonate de sodium ou bicarbonate de soude [144-55-8] = $NaHCO_3$, et le dihydrogénocarbonate de sodium = $Na(HCO_3)_2$.
Selon l'invention, le ou les agents (bi)carbonates utilisés représentent de préférence, de 0.001% à 10% en poids du poids total de la ou des compositions contenant le ou les agents (bi)carbonates et encore plus préférentiellement de 0,005% à 5% en poids.

*<u>v) l'eau:</u>*

Selon une mode de réalisation de l'invention, de l'eau est de préférence incluse dans le procédé de l'invention. Elle peut provenir de l'humidification des fibres kératiniques et/ou de la ou des compositions comprenant les composés i) à iv) tels que définis précédemment ou d'une ou plusieurs autres compositions.
De préférence l'eau provient au moins d'une composition comprenant au moins un composé choisi parmi i) à iv) tels que définis précédemment.

*<u>vi) compositions cosmétiques:</u>*

Un objet de l'invention concerne une composition cosmétique de coloration comprenant :

i) un ou plusieurs dérivé(s), synthétique(s) ou naturel(s), seuls ou en mélanges, choisis parmi les dérivés de formule (I) et (II) ou leurs formes mésomères, leurs stéréoisomères, leurs sels d'addition à un acide ou de base cosmétiquement acceptable, ainsi que les hydrates ;
ii) un ou plusieurs sel(s) métallique(s) ;
iii) du peroxyde d'hydrogène ou un ou plusieurs système(s) générateur(s) de peroxyde d'hydrogène ; et
iv) un ou plusieurs (bi)carbonate(s) ou un ou plusieurs système(s) générateur(s) de (bi)carbonate(s).

Les compositions cosmétiques selon l'invention sont cosmétiquement acceptables. Elles peuvent se trouver sous formes pulvérulentes, ou non.
Ces compositions peuvent comprendre un support de coloration qui contient généralement de l'eau ou un mélange d'eau et d'un ou plusieurs solvants organiques ou un mélange de solvants organiques.
Selon un mode particulier de l'invention, la composition cosmétique de coloration selon l'invention contient également de l'eau.

*Les Solvants organiques:*

A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, l'hexylène glycol, ainsi que les alcools aromatiques comme l'alcool benzylique ou le phénoxyéthanol.
Les solvants organiques sont présents dans des proportions de préférence comprises entre 1 et 40 % en poids environ par rapport au poids total de la composition tinctoriale, et encore plus préférentiellement entre 5 et 30 % en poids environ.

*Les Adjuvants:*

La ou les compositions du procédé de coloration conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiant.

Lesdits adjuvants sont choisis de préférence parmi des agents tensioactifs tels que des tensioactifs anioniques, non ioniques ou leurs mélanges et des agents épaississants minéraux ou organiques.

Les adjuvants ci-dessus sont en général présents en quantité comprise pour chacun d'eux entre 0,01 et 40 % en poids par rapport au poids de la composition, de préférence entre 0,1 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition ou aux composition(s) utiles dans le procédé de coloration conforme à l'invention ne soient pas, ou substantiellement pas, altérées par la ou les adjonctions envisagées.

*Les Colorants additionnels:*

La composition colorante comprenant les ingrédients i) à iv) tels que définis précédemment ainsi que le procédé mettant en oeuvre les ingrédients i) à iv) tels que définis précédemment, peuvent contenir en outre, ou mettre en oeuvre en outre, un ou plusieurs colorants directs additionnels.

Ces colorants directs sont par exemple choisis parmi ceux classiquement utilisés en coloration directe, et parmi lesquels on peut citer tous les colorants aromatiques et/ou non aromatiques d'utilisation courante tels que les colorants directs nitrés benzéniques neutres, acides ou cationiques, les colorants directs azoïques neutres, acides ou cationiques, les colorants directs naturels autres que les orthodiphénols, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les colorants fluorescents.

Parmi les colorants directs naturels, on peut citer la lawsone, la juglone, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Le ou les colorants directs alors dans la ou les compositions du procédé de coloration selon l'invention, ou de la composition selon l'invention, représentent de préférence, de 0.001% à 10% en poids environ du poids total de la ou des compositions mises en oeuvre et encore plus préférentiellement de 0.05% à 5% en poids environ.

La ou les compositions cosmétiques comprenant les ingrédients i), ii), iii) et iv) tels que définis précédemment, peuvent comprendre en outre une ou plusieurs bases d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

La ou les bases d'oxydation présentes dans la ou les compositions sont en général présentes chacune en quantité comprise entre 0,001 à 10 % en poids du poids total de la ou des compositions tinctoriales.

La ou les composition(s) cosmétique(s) de l'invention peuvent se présenter sous des formes galéniques diverses, telles qu'une poudre, une lotion, une mousse, une crème, un gel ou sous tout autre forme appropriée pour réaliser une teinture des fibres kératiniques. Elles peuvent également être conditionnées en flacon pompe sans propulseur ou sous pression en flacon aérosol en présence d'un agent propulseur et former une mousse.

*pH de la ou des composition(s)*

Selon un mode particulier de l'invention le pH de la ou des compositions aqueuses contenant iv) le ou les (bi) carbonates est supérieur à 7 et de préférence compris entre 8 et 12. Particulièrement compris entre 8 et 10.

Si le ou les compositions, ne contiennent pas de (bi)carbonates, le pH de la ou des compositions contenant

le peroxyde d'hydrogène ou un système générateur de peroxyde d'hydrogène, est de préférence inférieur à 7, plus particulièrement compris entre 1 et 5.

De préférence la ou les compositions contenant i) le ou les composés de formule (I) ou (II) de l'invention et ne contenant pas de (bi)carbonates sont à pH intérieur à 7 et de préférence comprise entre 3 et 6,5.

Selon un mode particulier de l'invention les compositions contenant ii) le ou les sels métallique et ne contenant pas de (bi)carbonates sont à pH inférieur à 7 et de préférence comprise entre 3 et 6,5.

Le pH de ces compositions peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants des compositions utilisées dans l'invention, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Une variante avantageuse est d'ajouter un agent alcalinisant à la ou les compositions du procédé de coloration contenant le ou les (bi)carbonates. Plus particulièrement, cet agent alcalin est choisi parmi l'ammoniaque, les carbonates alcalins, les alcanolamines telles que les mono-, di- et triéthanolamines ainsi que leurs dérivés, les hydroxydes de sodium ou de potassium et les composés de formule (IV) suivante :

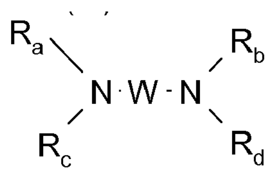
(IV)

Formule (IV) dans laquelle W est un reste propylène éventuellement substitué par un groupement hydroxy ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

*vii) Procédé de coloration en une ou plusieurs étapes*

Selon un mode de réalisation particulier de l'invention le procédé de coloration est réalisé, en une ou plusieurs étapes, par application sur les fibres kératiniques d'une ou de plusieurs compositions cosmétiques contenant, ensemble ou séparément, dans la ou les dites compositions, les ingrédients suivants:

i) un ou plusieurs dérivé(s), synthétique(s) ou naturel(s), seuls ou en mélanges, choisis parmi les dérivés de formule (I) et (II) ou leurs formes mésomères, leurs stéréoisomères, leurs sels d'addition à un acide ou de base cosmétiquement acceptable, ainsi que les hydrates ,
ii) un ou plusieurs sel(s) métallique(s), de préférence choisis parmi les sels de Mn et de Zn,
iii) du peroxyde d'hydrogène ou un ou plusieurs système(s) générateur(s) de peroxyde d'hydrogène et
iv) un ou plusieurs (bi)carbonate(s).

Avantageusement au moins une desdites compositions contenant, ensemble ou séparément, les ingrédients i) à iv) est aqueuse.

Le temps de pause entre les étapes d'application des compositions comprenant le ou les ingrédients i), ii), iii) et/ou iv) est fixé entre 3 et 120 minutes, préférentiellement entre 10 et 60 minutes et plus particulièrement entre 15 et 45 minutes.

Plus particulièrement, dans le procédé de l'invention le ou les composé(s) iv) se trouvent :

- soit en mélange avec les ingrédients i), ii) et iii)
- soit appliqué séparément après application d'une composition cosmétique comprenant les ingrédients i), ii) et iii) ; ou alors
- soit appliqué ensemble avec l'ingrédient iii) après application d'une composition cosmétique comprenant les ingrédients i) et ii).

Un mode de réalisation particulier de l'invention concerne les procédés de coloration en une ou deux étapes.

Selon un mode particulier de l'invention le procédé de coloration des fibres kératiniques est réalisé en une seule étape par l'application sur les fibres kératiniques d'une composition cosmétique colorante comprenant i), ii), iii) et iv) tels que définis précédemment.

Le temps de pose est généralement fixé entre 3 et 120 minutes et préférentiellement entre 10 et 60 minutes et plus préférentiellement entre 15 et 45 minutes.

Selon un autre mode de réalisation particulier de l'invention, le procédé de coloration de fibres kératiniques se fait en deux étapes.

Dans une première variante de procédé en deux étapes la première étape consiste à appliquer sur les dites fibres une composition cosmétique comprenant les ingrédients i), ii), iii) tels que définis précédemment, puis dans une deuxième étape une composition cosmétique comprenant l'ingrédient iv) tel que défini précédemment est appliquée sur lesdites fibres, étant entendu qu'au moins une des deux compositions cosmétiques est aqueuse.

Dans une deuxième variante de procédé de coloration de fibres kératiniques en deux étapes, la première étape consiste à appliquer sur les dites fibres une composition comprenant les ingrédients i) et ii), tels que définis précédemment, puis dans une deuxième étape une seconde composition cosmétique comprenant les ingrédients iii) et iv) tels que définis précédemment est appliquée sur lesdites fibres.

Selon un procédé de coloration particulier de l'invention, ledit procédé est réalisé en au moins deux étapes se terminant par le traitement des fibres kératiniques avec l'ingrédient iv) et peut être suivi d'étapes de post- traitement tel que d'un shampooinage à l'aide de shampoing classique, d'un rinçage par exemple à l'eau et/ou du séchage des fibres kératiniques par traitement thermique tel que défini ci après; étant entendu que ledit procédé ne fait pas intervenir de rinçage intermédiaire juste avant l'étape qui met en oeuvre l'ingrédient iv).

Préférentiellement, le procédé de coloration selon l'invention est réalisé en deux étapes dont la première étape est d'appliquer sur les fibres kératiniques les ingrédients i) et ii) ensemble puis dans une deuxième étape d'appliquer ensemble les ingrédients iii) et iv) ou dans une première étape d'appliquer ensemble les ingrédients i), ii) et iii) puis dans une deuxième étape d'appliquer iv). Ces procédés peuvent être suivis d'étapes de post-traitement tel que le rinçage par exemple à l'eau, le shampooinage avec un shampoing classique et/ou le séchage des fibres kératiniques. Préférentiellement, le procédé selon l'invention en au moins deux étapes ne fait pas intervenir de rinçage intermédiaire entre la première et la deuxième étape i.e. entre l'application du mélange des ingrédients i), ii), iii) et iv) ou entre l'application du mélange i), ii) et du mélange iii), iv).

Selon un procédé particulièrement avantageux les fibres kératiniques sont juste avant l'étape qui met en oeuvre l'ingrédient iv) :

- a) soit essuyé mécaniquement tel que décrit ci après,
- b) soit séchées par la chaleur avec un traitement thermique tel que décrit ci après,
- c) soit non rincées c'est-à-dire que les étapes se font successivement. De façon préférée entre la première et la deuxième étape du procédé de coloration de l'invention les fibres sont :
    - a) soit essuyé mécaniquement tel que décrit ci après,
    - b) soit séchées par la chaleur avec un traitement thermique tel que décrit ci après,
    - c) soit non rincées c'est-à-dire que les étapes 1 et 2 se font successivement.

Selon un procédé particulièrement préféré de l'invention juste avant l'étape qui met en oeuvre l'ingrédient iv) les fibres sont a) essuyées mécaniquement.

Plus préférentiellement entre la première et deuxième étape les fibres sont essuyées préférentiellement à l'aide d'une serviette ou d'un papier absorbant, ou sont séchées par la chaleur avec un traitement thermique à une température comprise particulièrement entre 60 et 220°C et de préférence entre 120 et 200°C.

Selon un autre mode particulier de l'invention entre l'application du mélange des ingrédients i), ii), iii) et iv) ou entre l'application du mélange i), ii) et du mélange iii), iv), les mèches sont rincées très rapidement, entre 1 seconde et 1 minute plus particulièrement entre 1 seconde et 30 seconde, et préférentiellement entre 2 et 5 secondes tel que 2 secondes, sous à l'eau d'un robinet ou douchette à jet intense. Cette étape de rinçage rapide est suivie d'un essuyage mécanique tel que décrit ci après.

Pour ces deux derniers procédés le temps de pause après application de la composition cosmétique pour la première étape est généralement fixé entre 3 et 120 minutes et préférentiellement entre 10 et 60 minutes et plus préférentiellement entre 15 et 45 minutes. Le temps de pause après application de la deuxième composition cosmétique pour la deuxième étape est généralement fixé entre 3 et 120 minutes et préférentiellement entre 3 et 60 minutes et plus préférentiellement entre 5 et 30 minutes.

Quelque soit le mode d'application, la température d'application est généralement comprise entre la température ambiante (15 à 25°C) et 80°C et plus particulièrement entre 15 et 45°C. Ainsi, on peut, avantageusement, après application de la composition selon l'invention, soumettre la chevelure à un traitement thermique par chauffage à une température comprise entre 30 et 60°C. Dans la pratique, cette opération peut être conduite au moyen d'un casque de coiffure, d'un sèche-cheveux, d'un dispensateur de rayons infrarouges et d'autres appareils chauffants classiques.

On peut utiliser, à la fois comme moyen de chauffage et de lissage de la chevelure, un fer chauffant à une

température comprise entre 60 et 220°C et de préférence entre 120 et 200°C.

Un mode particulier de l'invention concerne un procédé de coloration qui est réalisé à température ambiante (25 °C).

Dans tous les modes particuliers et variantes des procédés précédemment décrits les compositions évoquées sont des compositions prêtes à l'emploi qui peuvent résulter du mélange extemporané de deux ou plusieurs compositions et notamment de compositions présentes dans des kits de teintures.

*viii) étape(s) d'essuyage mécanique et/ou séchage :*

Selon un mode particulier de l'invention, le procédé de coloration des fibres kératiniques comprend au moins une étape intermédiaire d'essuyage mécanique des fibres et/ou de séchage et/ou non rincées. Les étapes d'essuyage mécanique et de séchage intermédiaire sont également appelées « non rincé maitrisé » pour se différencier du « rinçage classique à grande eau » et du « non rincé ».

Par essuyage mécanique des fibres on entend le frottement d'un objet absorbant sur les fibres et le retrait physique par l'objet absorbant du surplus d'ingrédient(s) n'ayant pas pénétré dans les fibres. L'objet absorbant peut être une pièce de tissu comme une serviette particulièrement éponge, un torchon, du papier absorbant tel que de l'essuie-tout.

Selon un procédé particulièrement avantageux de l'invention, l'essuyage mécanique est effectué sans séchage total de la fibre, laissant la fibre humide.

Par séchage on entend l'action d'évaporer des solvants organiques et/ou de l'eau se trouvant dans une ou plusieurs compositions utilisées dans le procédé de l'invention, comprenant ou non un ou plusieurs ingrédients i à iv) tels que définis précédemment. Le séchage peut se faire par source thermique (convection, conduction ou rayonnement) en envoyant par exemple un courant gazeux chaud tel que l'air nécessaire à l'évaporation du ou des solvants. A titre de source thermique on peut citer un sèche-cheveux, de casques à cheveux, d'un fer à lisser les cheveux, d'un dispensateur de rayons infrarouges et d'autres appareils chauffants classiques.

*ix) Dispositif ou « kit » de teinture :*

Un autre objet de l'invention est un dispositif à plusieurs compartiments ou « kit » de teinture. De façon avantageuse, ce kit comporte de 2 à 5 compartiments contenant de 2 à 5 compositions dans lesquels sont réparties les ingrédients

i) un ou plusieurs dérivé(s), synthétique(s) ou naturel(s), seuls ou en mélanges, choisis parmi les dérivés de formule (I) et (II) ou leurs formes mésomères, leurs stéréoisomères, leurs sels d'addition à un acide ou de base cosmétiquement acceptable, ainsi que les hydrates ,
ii) un ou plusieurs sel(s) métallique(s),
iii) du peroxyde d'hydrogène ou un ou plusieurs système(s) générateur(s) de peroxyde d'hydrogène, et
iv) un ou plusieurs (bi)carbonate(s),

lesdites compositions étant aqueuses ou pulvérulentes, avec particulièrement au moins une de ces compositions étant aqueuse.

Selon une première variante le kit comporte cinq compartiments, les quatre premiers compartiments comprenant respectivement les ingrédients en poudres i), ii), iii) et iv) tels que définis précédemment et dans le cinquième compartiment contenant une composition aqueuse tel que de l'eau. Dans ce cas le ou les composés iii) sont des précurseurs de peroxyde d'hydrogène.

Une autre variante concerne un kit à quatre compartiments dont l'un d'entre eux contient une composition aqueuse, et dont les compartiments comprennent un ingrédient i) à iv) tels que définis précédemment.

Dans une autre variante le dispositif comprend quatre compartiments : un premier compartiment comprenant une composition cosmétique contenant i) tel que défini précédemment, un deuxième compartiment comprenant ii) tel que défini précédemment, un troisième compartiment comprenant iii) tel que défini précédemment et un quatrième compartiment contenant iv) tel que défini précédemment.

Un autre mode de réalisation préféré concerne un dispositif comprenant trois compartiments :

(a) un premier compartiment contient une composition renfermant :

i) un ou plusieurs dérivé(s), synthétique(s) ou naturel(s), seuls ou en mélanges, choisis parmi les dérivés de formule (I) et (II) ou leurs formes mésomères, leurs stéréoisomères, leurs sels d'addition à un acide ou de base cosmétiquement acceptable, ainsi que les hydrates ; et

(b) un deuxième compartiment contient une composition renfermant :

ii) un ou plusieurs sel(s) métallique(s),
iii) du peroxyde d'hydrogène ou un ou plusieurs système(s) générateur(s) de peroxyde d'hydrogène ;

(c) un troisième compartiment contient iv) un ou plusieurs (bi)carbonate(s) ou un ou plusieurs système(s) générateur (s) de (bi)carbonate(s)

Dans cet autre mode de réalisation, au moins une des trois compositions est aqueuse et le ou les dérivés de formule (I) et (II) peuvent être sous forme de poudre.
On peut aussi avoir un kit à trois compartiments, le premier a) contenant une composition comprenant i) et ii) tels que défini précédemment, le second b) contenant une composition comprenant iii) tel que défini précédemment et le troisième c) contenant une composition comprenant iv) tel que défini précédemment. Dans cet autre kit au moins une des compositions est aqueuse.
Selon un mode particulier de l'invention, le kit comporte deux compartiments : un premier compartiment comprenant une composition contenant i), ii) et iii) tels que définis précédemment et un deuxième compartiment contenant iv) tel que défini précédemment.
Parmi les kits à deux compartiments sont également possibles les kits qui contiennent dans un premier compartiment une composition comprenant i), ii) et iv) tels que définis précédemment et dans un deuxième compartiment une composition comprenant respectivement iii) tel que défini précédemment.
Dans ces deux variantes de kit à deux compartiments la première composition contenue dans le premier compartiment comprenant soit i), ii) et iii) ou i), ii) et iv) est sous forme de poudre.
Selon une variante, le dispositif selon l'invention, comprend, en outre, une composition supplémentaire (c) comprenant un ou plusieurs agents traitants.
Les compositions du dispositif selon l'invention sont conditionnées dans des compartiments distincts, accompagnés, éventuellement, de moyens d'application appropriés, identiques ou différents, tels que les pinceaux, les brosses ou les éponges.
Le dispositif mentionné ci-dessus peut également être équipé d'un moyen permettant de délivrer sur les cheveux le mélange souhaité, par exemple tel que les dispositifs décrits dans le brevet FR 2 586 913.

## I) EXEMPLES DE COLORATION

Les compositions suivantes ont été préparées :

| **Composition A** | **A1** | **A2** | **A3** |
|---|---|---|---|
| Hématoxyline | 5g | - | - |
| Extrait de bois de campêche | - | 5g | - |
| Extrait de bois du brésil | - | - | 5g |
| Héxylène glycol | 5g | 5g | 5g |
| Lauryl ether sulfate de sodium (70% en MA dans l'eau) | 3,75g | 3,75g | 3,75g |
| Chlorure de manganèse tétrahydraté (soit 0,01 % en poids d'équivalent métal $Mn^{2+}$) | 0,036g | - | - |
| Gluconate de manganèse (soit 0,01 % en poids d'équivalent métal $Mn^{2+}$) | - | 0,081 g | - |
| Pyrrolidone carboxylate de manganèse (soit 0,01% en poids d'équivalent métal $Mn^{2+}$) | - | - | 0,062g |
| Peroxyde d'hydrogène | 1,2g | 1,2g | - |
| Polyvinylpyrrolidone/$H_2O_2$ (soit 1,2% en poids d'équivalent de peroxyde d'hydrogène) | - | - | 6g |
| Acide citrique ou hydroxyde de sodium | qsp pH 5 | qsp pH 5 | qsp pH 5 |
| Eau déminéralisée | qsp 100g | qsp 100g | qsp 100g |

La composition **A** est appliquée sur des mèches de cheveux secs à 90% de blancs naturels et à 90% de blancs permanentés avec un rapport de bain de 5 g de formule pour 1g de cheveux. On laisse ensuite poser pendant 30 minutes à une température de 50°C.

A l'issu, les cheveux imprégnés de la première composition sont essuyés à l'aide d'une serviette de papier absorbant pour enlever l'excédant de formule.

| **Composition B** | **b1** |
|---|---|
| Bicarbonate de sodium $NaHCO_3$ | 2,6g |
| Carbomer | 1g |
| Monoéthanolamine | qsp pH 9 |
| Eau déminéralisée | qsp 100g |

La composition B est ensuite appliquée sur les cheveux avec un rapport de bain de 4g pour 1g de mèche ; le temps de pose est de 10 minutes à température ambiante. Au bout de quelques minutes une coloration très intense apparaît. Les cheveux sont ensuite rincés à l'eau, lavés avec un shampooing classique et séchés au casque.

**Résultats colorimétriques :**

La coloration des cheveux est évaluée visuellement et lue au spectrocolorimètre Minolta (CM3600d, illuminant D65, angle 10°, valeurs SCI) pour les mesures colorimétriques $L^*$, $a^*$, $b^*$.

Dans ce système $L^*$ $a^*$ $b^*$, $L^*$ représente l'intensité de la couleur, $a^*$ indique l'axe de couleur vert/rouge et $b^*$ l'axe de couleur bleu/jaune. Plus la valeur de L est faible, plus la couleur est foncée ou très intense. Plus la valeur de $a^*$ est élevée plus la nuance est rouge et plus la valeur de $b^*$ est élevée plus la nuance est jaune.

La variation de coloration entre les mèches colorées de cheveux blancs naturels/permanentés non traités (témoin) et après traitement sont définis par ($\Delta E^*$) selon l'équation suivante :

$$\Delta E^* = \sqrt{(L^* - L_o^*)^2 + (a^* - a_o^*)^2 + (b^* - b_o^*)^2}$$

Dans cette équation, $L^*$, $a^*$ et $b^*$ représentent les valeurs mesurées après coloration des cheveux naturels/permanentés à 90% de blancs et $L^*_0$, $a_0^*$ et $b_0^*$ représentent les valeurs mesurées des cheveux naturels/permanentés à 90% non traités.

Plus la valeur de $\Delta E$ est importante, plus la différence de couleur entre les mèches témoins et les mèches colorées est importante.

La coloration est très tenace aux lavages et à la lumière.

la coloration des cheveux est évaluée visuellement et lue au spectrocolorimètre Minolta (CM3600d, illuminant D65, angle 10°, valeurs SCI) pour les mesures colorimétriques $L^*$, $a^*$, $b^*$.

La coloration est très tenace aux lavages et à la lumière.

| **Exemples** (sur cheveux à 90% de blancs naturels) | **Témoin** (cheveux non traités) | **1** | **2** | **3** |
|---|---|---|---|---|
| Composition (Ai) Etape 1 | - | A1 | A2 | A3 |
| Composition (Bi) Etape 2 | - | B1 | B1 | B1 |
| Nuances sur cheveux | - | violine intense | violine intense | rouge-rose |
| L* | 55,6 | 20,34 | 23,1 | 35,4 |
| a* | 0,63 | 4,75 | 6,58 | 17,12 |
| b* | 14,36 | 0,9 | 0,23 | 10,08 |
| ΔE* | - | 37,97 | 35,94 | 26,42 |
| ΔL* | - | -35,26 | -32,51 | -20,2 |
| Δa* | - | 4,13 | 5,95 | 16,49 |
| Δb* | - | -13,46 | -14,12 | -4,28 |

| **Exemples** (sur cheveux à 90% de blancs naturels permanentés) | **Témoin** (cheveux non traités) | **4** | **5** | **6** |
|---|---|---|---|---|
| Composition (Ai) Etape 1 | - | A1 | A2 | A3 |
| Composition (Bi) Etape 2 | - | B1 | B1 | B1 |
| Nuances sur cheveux | - | violine très intense | violine très intense | rouge-rose intense |
| L* | 55,19 | 18,58 | 18,69 | 27,78 |
| a* | 0,7 | 5,31 | 2,57 | 21,37 |
| b* | 13,32 | 1,76 | -0,11 | 9,43 |
| ΔE* | - | 38,67 | 38,94 | 34,56 |
| ΔL* | - | -36,61 | -36,51 | -27,42 |
| Δa* | - | 4,61 | 1,86 | 20,67 |
| Δb* | - | -11,56 | -13,43 | -3,89 |

Il apparaît des tableaux ci-dessus que les mèches de cheveux blancs naturels ou permanentés traités avec la composition selon l'invention permet de colorer de façon très chromatique et intense et ce sur fibres kératiniques naturelles ou permanentées.

## II) EXEMPLES COMPARATIFS

| **Composition A'** | **A'1 Invention** | **A'2 comparatif** |
|---|---|---|
| Hématoxyline | ($1{,}38.10^{-3}$ mole) | - |
| Catéchine | - | ($1{,}38.10^{-3}$ mole) |
| Héxylène glycol | 5g | 5g |
| Lauryl ether sulfate de sodium (70% en MA) | 3,75g | 3,75g |
| Chlorure de manganèse tétrahydraté (soit 0,01% en poids d'équivalent métal $Mn^{2+}$) | 0,036g | 0,036 g |
| Peroxyde d'hydrogène | 1,2g | 1,2g |
| Acide citrique ou hydroxyde de sodium | qsp pH 5 | qsp pH 5 |
| Eau déminéralisée | qsp 100g | qsp 100g |

La composition **A'** est appliquée sur des mèches de cheveux secs à 90% de blancs naturels ou permanentés avec un rapport de bain de 5 g de formule pour 1g de cheveux. On laisse ensuite poser pendant 30 minutes à une température de 50 °C.

A l'issu du temps de pose, les cheveux imprégnés de la première composition sont essuyés à l'aide d'une serviette de papier absorbant pour enlever l'excédant de formule.

| **Composition B** | **B'1** |
|---|---|
| Bicarbonate de sodium $NaHCO_3$ | 2,6g |
| Carbomer | 1 g |
| Monoéthanolamine | qsp pH 9 |
| Eau déminéralisée | qsp 100g |

La composition B' est ensuite appliquée sur les cheveux avec un rapport de bain de 4 g pour 1g de mèche ; le temps de pose est de 10 minutes à température ambiante. Au bout de quelques minutes une coloration très intense apparaît.

**Résultats colorimétriques**

Après un rinçage, un shampooing et un séchage au casque des mèches, la coloration des cheveux est évaluée visuellement et lue au spectrocolorimètre Minolta CM3600d (illuminant D65, angle 10°, composante spéculaire incluse) pour les mesures colorimétriques $L^*$, $a^*$, $b^*$.

**Chromaticité : C***

La chromaticité dans le système CIE $L^*$, $a^*$, $b^*$ est calculée selon l'équation suivante :

$$C^* = \sqrt{a^{*2} + b^{*2}}$$

Plus la valeur de $C^*$ est élevée plus la coloration obtenue est chromatique

| **Exemples** (sur cheveux à 90% de blancs naturels) | **7** | **8** |
|---|---|---|
| Composition (A'i) Etape 1 | A'1 | A'2 |
| Composition (B'i) Etape 2 | B'1 | B'1 |
| Nuances sur cheveux | violine intense | cuivré intense |
| Chromaticité (C*) | 32,50 | 11,36 |
| | | |
| **Exemples** (sur cheveux à 90% de blancs permanentés) | **7** | **8** |
| Composition (A'i) Etape 1 | A'1 | A'2 |
| Composition (B'i) Etape 2 | B'1 | B'1 |
| Nuances sur cheveux | violine intense | cuivré intense |
| Chromaticité (C*) | 28,10 | 11,98 |

Il apparaît des tableaux ci-dessus que les mèches de cheveux à 90% blancs naturels ou permanentés traités avec la composition selon l'invention permet de colorer de façon significativement plus chromatique que la composition selon le comparatif (voir A'1+B'1 exemple 7 vs. A'2+B'1 exemple 8.

## Revendications

1. Procédé de coloration des fibres kératiniques, dans lequel lesdites fibres sont traitées par :

i) un ou plusieurs composé(s), synthétique(s) ou naturel(s), seuls ou en mélanges, choisis parmi les composés de formule (I) et (II) et leurs formes mésomères, leurs stéréoisomères, leurs sels d'addition à un acide ou de base cosmétiquement acceptable, ainsi que les hydrates ;

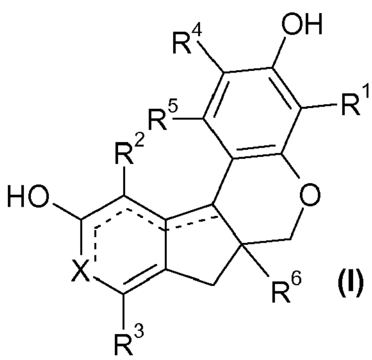
**(I)**
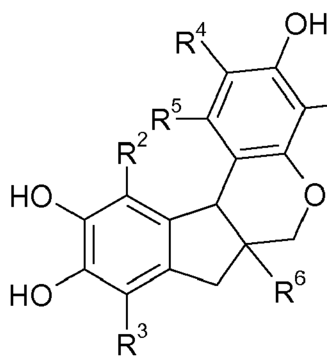
**(II)**

formules **(I)** et **(II)** dans lesquelles :

- ---- représente une liaison carbone - carbone simple ou double conjuguée,
- X représente un groupement :

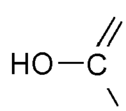
ou
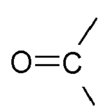

- $R^1$, $R^2$, $R^3$, $R^4$, $R^5$ et $R^6$, identiques ou différents, représentent un atome d'hydrogène, un groupe hydroxy, un groupe alkyle éventuellement substitué, alcoxy éventuellement substitué, ou un groupe acyloxy éventuellement substitué

ii) un ou plusieurs sel(s) métallique(s) ;
iii) du peroxyde d'hydrogène ou un ou plusieurs système(s) générateur(s) de peroxyde d'hydrogène ; et
iv) un ou plusieurs (bi)carbonate(s)..

**2.** Procédé de coloration selon la revendication précédente dans lequel l'ingrédient i) est choisi parmi les dérivés naturel(s).

**3.** Procédé de coloration selon la revendication 1 ou 2 dans lequel le ou les composés de formule (I) ou (II) comprennent un radical $R^6$ qui représente un groupe hydroxy.

**4.** Procédé de coloration selon une quelconque des revendications précédentes dans lequel le ou les composés de formule (I) ou (II) comprennent $R^1$ qui représente un atome d'hydrogène ou un groupe hydroxy.

**5.** Procédé de coloration selon une quelconque des revendications précédentes dans lequel l'ingrédient i) est un composé choisi parmi les composés hématoxyline, braziline, hématéine et braziléine et leurs formes mésomères, leurs stéréoisomères, leurs sels d'addition à un acide ou de base cosmétiquement acceptable, ainsi que les hydrates

| | |
|---|---|
| 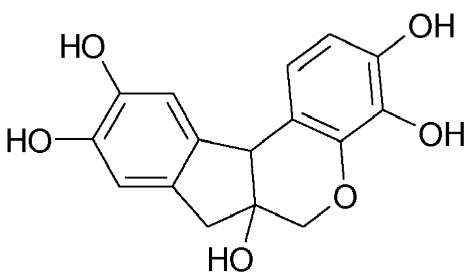<br>**Hématoxyline** | 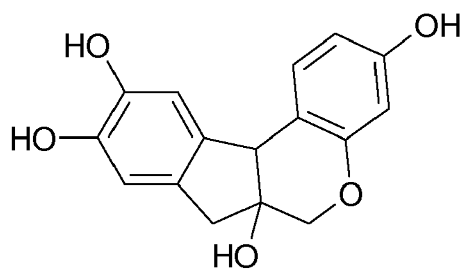<br>**Braziline** |
| 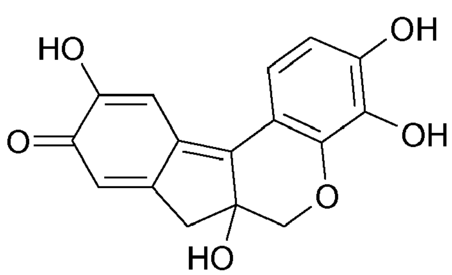<br>**Hématéine** | 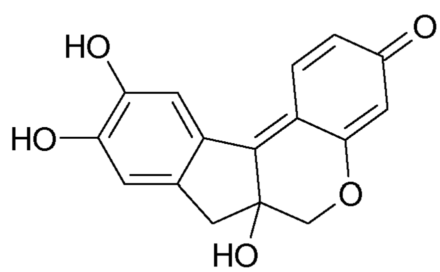<br>**et Braziléine** |

**6.** Procédé de coloration selon une quelconque des revendications précédentes dans lequel l'ingrédient i) est un composé choisi parmi les composés de formule (I).

**7.** Procédé de coloration selon une quelconque des revendications précédentes dans lequel l'ingrédient i) est choisi parmi les extraits végétaux suivants *Haematoxylon campechianum, Haematoxylon brasiletto, Caesalpinia echinata, Caesalpinia sappan, Caesalpinia spinosa et et Caesalpina Brasiliensis.*

**8.** Procédé de coloration selon une quelconque des revendications précédentes dans lequel le ou les sel(s) métallique (s) contenu(s) ii) sont choisis parmi les oxydes de Mn et Zn.

**9.** Procédé selon une quelconque des revendications 1 à 7 dans lequel les sels de Mn et Zn, sont choisis parmi les halogénures, les sulfates, phosphates, nitrates et perchlorates, les sels d'acides carboxyliques et les sels polymériques ainsi que leurs mélanges.

**10.** Procédé de coloration selon une quelconque des revendications précédentes dans lequel l'ingrédient iii) contient du peroxyde d'hydrogène ou un système générateur de peroxyde d'hydrogène choisi parmi :

a) le peroxyde d'urée ;
b) les complexes polymériques pouvant libérer du peroxyde d'hydrogène ;
c) les oxydases produisant du peroxyde d'hydrogène en présence d'un substrat adéquat;
d) les peroxydes de métaux générant dans l'eau du peroxyde d'hydrogène;
e) les perborates ; ou
f) les percarborates.

**11.** Procédé de coloration selon une quelconque des revendications précédentes dans lequel l'ingrédient iv) contient du ou des (bi)carbonate(s) alcalins ou alcalino-terreux.

**12.** Procédé selon une quelconque des revendications précédentes en une étape consistant à appliquer sur les fibres kératiniques une composition cosmétique comprenant i), ii), iii), et iv) tels que définis dans une quelconque des revendications précédentes.

**13.** Procédé de coloration selon une quelconque des revendications 1 à 11 en deux étapes consistant dans la première étape à appliquer sur les fibres kératiniques une composition cosmétique comprenant les ingrédients i), ii) et iii) tels que définis dans une quelconque des revendications 1 à 10, puis dans une deuxième étape consistant à appliquer une composition cosmétique comprenant iv) tel que défini dans les revendications 1 ou 11.

**14.** Procédé de coloration selon une quelconque des revendications 1 à 11 en deux étapes consistant dans la première étape à appliquer sur les fibres kératiniques une composition comprenant les ingrédients i) et ii) tels que définis dans une quelconque des revendications 1 à 9 puis dans une deuxième étape consistant à appliquer une composition comprenant iii) et iv) tels que définis dans les revendications 1, 10 ou 11.

**15.** Procédé de coloration selon une quelconque des revendications 1 à 14 dans lequel les fibres kératiniques sont juste avant l'étape qui met en oeuvre l'ingrédient iv) :

a) soit essuyées mécaniquement,
b) soit séchées par la chaleur avec un traitement thermique,
c) soit non rincées c'est-à-dire que les étapes se font successivement

plus particulièrement juste avant l'étape qui met en oeuvre l'ingrédient iv) les fibres sont essuyées mécaniquement.

**16.** Procédé selon l'une quelconque des revendications précédentes dans lequel une au moins des compositions comprenant au moins un des ingrédients i) à iv) est aqueuse.

**17.** Composition cosmétique de coloration comprenant :

- un ou plusieurs ingrédient i) tels que définis dans une quelconque des revendications 1 à 7;
- un ou plusieurs ingrédients ii) tels que définis dans une quelconque des revendications 1, 8 et 9 ;
- un ou plusieurs ingrédients iii) tels que définis dans une quelconque des revendications 1 et 10 ; et
- un ou plusieurs ingrédient iv) tels que définis dans les revendications 1 et 11.

**18.** Dispositif à plusieurs compartiments comprenant de 2 à 5 compartiments contenant de 2 à 5 compositions dans lesquels sont réparties les ingrédients i), ii), iii) et iv) tels que définis dans une quelconque des revendications 1 à 11.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2814943
- FR 2814945
- FR 2814946
- FR 2814947
- US 5008093 A
- US 3376110 A
- US 5183901 A
- FR 2586913